# EUROPEAN PATENT SPECIFICATION

(11) **EP 3 125 856 B1**
(45) Date of publication and mention of the grant of the patent: **03.07.2019**
(21) Application number: 15711550.2
(22) Date of filing: 24.03.2015
(51) Int. Cl.: A61K 8/25, A61K 8/34, A61K 8/73, A61Q 17/04, A61K 8/92, A61K 8/02, A61K 8/31, A61K 8/06, A61K 8/55

(54) **TOPICAL COMPOSITIONS**
TOPISCHE ZUSAMMENSETZUNGEN
COMPOSITIONS TOPIQUES

(30) Priority: 31.03.2014 EP 14162823
(43) Date of publication of application: 08.02.2017
(73) Proprietor: DSM IP Assets B.V., 6411 TE Heerlen (NL)
(72) Inventor: HUEBER, Aline, CH-4303 Kaiseraugst (CH); JANSSEN, Anne, CH-4303 Kaiseraugst (CH); SAECKER, Christine, CH-4303 Kaiseraugst (CH)
(74) Representative: Berg, Katja
(86) International application number: PCT/EP2015/056262
(87) International publication number: WO 2015/150169

(56) References cited:
- JP-A- 2002 029 961
- JP-A- 2007 070 232
- US-A1- 2005 031 655
- Fuji Silysia: "Sylysia 550", Fuji Silysia , XP002728023, Retrieved from the Internet: URL:http://www.viacheminc.com/wp-content/u ploads/Tech-Data-Sheet-SYLYSIA-550.pdf [retrieved on 2014-07-30]
- DATABASE GNPD [Online] MINTEL; April 2012 (2012-04), "Face & Décolleté Cream SPF 50+", XP002728024, Database accession no. 1776424
- Dsm: "Valvance touch 210", , 21 October 2013 (2013-10-21), pages 1-3, XP055408285, Retrieved from the Internet: URL:http://www.essence-plus.com/essence-pl us689/program_download/good/20161108140437 5480.pdf [retrieved on 2017-09-20]

## Description

The present invention relates to specific topical composition according to the claims comprising glycerin, microcrystalline cellulose and silica beads having a particle size Dᵥ0 of greater 0.3 µm, a Dᵥ100 of less than 35 µm, a Dᵥ50 selected in the range of 9 to 15 µm and an oil absorption capacity selected in the range of 1.2-2.5 cc/g. Furthermore the invention relates to the use of such beads in combination with microcrystalline cellulose to ameliorate the short and long-term sensory properties of such topical composition comprising glycerin.

Glycerin is a humectant that is commonly added to cosmetic compositions such as sun care products. However, incorporated into topical compositions, glycerin can impart a very sticky feeling in particular when used in higher amounts. This drawback is even more pronounced in sun-care products (sunscreens) which due to the presence of oil soluble UV-filters already tend to be sticky.

JP2002/029961A discloses highly porous silica particles having a water-absorbing ability of ≥400 mL/100g and an apparent average diameter of 1 to 100 µm to reduce stickiness of W/O emulsions comprising glycerin and microcrystalline wax.

US2005/031655 relates to sunscreen compositions that contain porous silica microspheres which are not greasy, sticky or oily, is however completely silent with respect to the use of microcrystalline cellulose or wax.

Thus, there is an ongoing need for substances which reduce the stickiness of glycerin in topical compositions such as in particular in sun care products comprising oil soluble UV-filter(s) and thus contribute to an overall improved skin feel.

Surprisingly it has been found that the addition of specific silica beads in combination with microcrystalline cellulose to certain topical composition comprising glycerin significantly reduced the stickiness of such compositions. Furthermore, the topical compositions exhibited a significantly enhanced absorbency and a superior dry touch.

Thus, the present invention relates to a topical composition comprising glycerin, microcrystalline cellulose and silica beads having a particle size Dᵥ0 of greater 0.3 µm, a Dᵥ100 of less than 35 µm, a Dᵥ50 selected in the range of 9 to 15 µm and an oil absorption capacity selected in the range of 1.2-2.5 cc/g according to the claims.

Another subject matter of the invention is directed to a method reducing the stickiness of a topical composition comprising glycerin, said method comprising the step of adding to the topical composition silica beads having a particle size Dᵥ0 of greater than 0.3 µm, a Dᵥ100 of less than 35 µm, a Dv50 selected in the range of 9 to 15 µm and an oil absorption capacity selected in the range of 1.2-2.5 cc/g in combination with microcrystalline cellulose according to claim 13 and appreciating the effect.

In a further embodiment the invention relates to the use of silica beads having a particle size Dᵥ0 of greater than 0.3 µm, a Dᵥ100 of less than 35 µm, a Dᵥ50 selected in the range of 9 to 15 µm and an oil absorption capacity selected in the range of 1.2-2.5 cc/g in combination with microcrystalline cellulose to reduce the stickiness of a topical composition comprising glycerin according to claim 12.

In an additional embodiment the invention relates to the use of silica beads having a particle size Dᵥ0 of greater than 0.3 µm, a Dᵥ100 of less than 35 µm, a Dᵥ50 selected in the range of 9 to 15 and an oil absorption capacity selected in the range of 1.2-2.5 cc/g in combination with microcrystalline cellulose to enhance dry touch feel of a topical composition comprising glycerin.

Another subject matter of the invention is directed to a method reducing the stickiness and/ or to enhance the absorbency of a topical composition comprising glycerin and silica beads having a particle size Dᵥ0 of greater than 0.3 µm, a Dᵥ100 of less than 35 µm, a Dv50 selected in the range of 9 to 15 µm and an oil absorption capacity selected in the range of 1.2-2.5 cc/g said method comprising the step of adding to the topical composition microcrystalline cellulose and appreciating the effect.

In another embodiment the invention relates to the use of microcrystalline cellulose to reduce the stickiness and/ or to enhance the absorbency of a topical composition comprising glycerin and silica beads having a particle size Dᵥ0 of greater than 0.3 µm, a Dᵥ100 of less than 35 µm, and a Dᵥ50 selected in the range of 9 to 15 µm and an oil absorption capacity selected in the range of 1.2-2.5 cc/g.

In all embodiments of the present invention, the amount of the silica beads is preferably selected in the range of 0.5 to 10 wt.-%, more preferably in the range of 1 to 5 wt.-%, and most preferably in the range of 2 to 4 wt.-% based on the total weight of the composition.

In all embodiments of the present invention, the total amount of the microcrystalline cellulose is preferably selected in the range of 0.1 to 10 wt.-%, more preferably in the range of 0.5 to 5 wt.-%, and most preferably in the range of 1 to 3 wt.-% based on the total weight of the composition. It is furthermore preferred that either microcrystalline cellulose alone (i.e. in the absence of microcrystalline wax) or microcrystalline cellulose in combination with microcrystalline wax is used in the topical compositions according to the present invention. Most preferably in all embodiments of the present invention microcrystalline cellulose is used in the absence of microcrystalline wax in the topical compositions as this results in the best performance in view of reduced stickiness and improved absorbency.

In all embodiments of the present invention, the amount of glycerin is preferably selected in the range of 1 to 20 wt.-%, more preferably in the range of 3 to 15 wt.-%, and most preferably in the range of 5 to 10 wt.-% based on the total weight of the composition.

The porous silica beads used according to the present invention may be prepared from sodium silicate by emulsion polymerization according to standard procedures such as e.g. via the sol-gel method. They silica beads may be used as such or may be further coated with a suitable coating agent such as e.g. a silicone oil. Suitable silicone oils include in particular non-volatile silicone oils such as dialkyl and alkyl aryl siloxanes as well as alkoxylated and / or aminated derivatives thereof, dihydroxypolydimethylsiloxanes and polyphenylalkylsiloxanes. Preferred silicone oils encompass dimethicone, dimethiconol, dimethicone copolyol, phenyl trimethicone, methicone, simethicone as well as mixtures thereof. Most preferably the silica beads are either uncoated or coated with dimethicone. In a preferred embodiment the silica beads used according to the present invention are uncoated.

The particle size of the beads according to the invention (in volume %) is determined by a Coulter LS13320 or Malvern Mastersizer 2000 according to standard methods in the art. In number % the average particle size Dₙ50 ranges from 9 to 15 µm.

In all embodiments of the present invention the oil absorption capacity of the silica beads is preferably selected in the range of 1.2 to 2.0 cc/g and most preferably in the range of 1.3 to 1.8 cc/g.

The oil absorption capacity refers to the weight of a specific oil absorbed by a material, determined by a specified method. It includes the oil absorption capacity of the dry particles existing between the inherent voids within and on the surface of the particles. The oil absorption capacity as referred to in the present invention is determined at 23°C by weighting 2g of the respective silica beads into a 20 ml beaker glass. Then liquid paraffin (Paraffinum Perliquidum PH.EUR. CAS 8042-47-5) is added. After addition of 4 to 5 drops of paraffin to the powder, mixing is performed using a spatula, and addition of paraffin is continued until conglomerates of oil and powder have formed. From this point, the paraffin is added one drop at a time and the mixture is then triturated with the spatula. The addition of oil is stopped when the loose and dry powder completely disappears and a highly viscous white to transparent homogeneous gel is obtained. The oil absorption capacity (cc/g) is then calculated by the volume of paraffin used (in cc) per g of silica beads.

Particularly suitable porous silica beads according to the present invention are e.g. obtainable as VALVANCE™ *Touch* 210 at DSM Nutritional Products Ltd Kaiseraugst.

Particularly suitable microcrystalline cellulose according to the present invention is the isolated, crystalline portion of cellulose fibers from wood pulp which can be used in colloidal (i.e. co-processed with a soluble hydrocolloid) or non-colloidal form. Particularly advantageous microcrystalline cellulose according to the present invention is available as Avicel range at FMC Biopolymer US. In a particular preferred embodiment, the microcrystalline cellulose is used in colloidal form having a particle size of <1 um, a rod-like particle shape. The initial viscosity is preferably selected in the range of 30 to 150 cps as a 1 to 3 % dispersion. Even more preferably, the microcrystalline cellulose has additionally a sieve fraction of +60mesh of not more than 0.1%wt and +325 mesh not more than 75 %wt. Most preferably, the microcrystalline cellulose is .Avicel 611 (INCI Microcrystalline Cellulose and Cellulose gum, having a particle size of <1 um, a rod-like particle shape and a sieve fraction +60mesh of not more than 0.1%wt and +325 mesh not more than 75 %wt. and an initial viscosity of 50 to 151 cps (2.6%dispersion))

Suitable microcrystalline waxes according to the present invention are of mineral origin and may be produced by de-oiling petrolatum, as part of the petroleum refining process. Microcrystalline waxes generally contain a high percentage of isoparaffinig (branched) hydrocarbons and naphtenic hydrocarbons. They are characterized by the fineness of its crystals in contrast to the larger crystal of paraffin wax. They consist of high molecular weight saturated aliphatic hydrocarbons. Particularly suitable microcrystalline wax according to the present invention is Paracera™ M available at Paramel or Kahlwax 1847 available at Kahl GmbH & Co..

In another embodiment of the present invention the topical compositions are light protective preparations (sunscreens) comprising additionally at least one oil soluble UV-filter substance.

The total amount of the at least one additional oil soluble UV-filter substance is preferably selected in the range of about 0.1 to 25 wt.-%, more preferably in the range of about 1 to 20 wt.-%, most preferably in the range of 5 to 15 wt.-% based on the total weight of the topical composition.

Particularly preferred oil soluble UV-filter substances encompass dibenzoylmethane derivatives such as butyl methoxydibenzoylmethane (e.g. commercially available as PARSOL® 1789 at DSM Nutritional Products Ltd), organopolysiloxanes functionalized with at least one UV-light absorbing group such as polysilicone-15 (e.g. commercially available as PARSOL® SLX at DSM Nutritional Products Ltd), diphenylacrylates such as octocrylene (e.g. commercially available as PARSOL® 340 at DSM Nutritional Products Ltd) and cinnamates such as octyl methoxycinnamate (e.g. commercially available as PARSOL® MCX at DSM Nutritional Products Ltd) as well as mixtures thereof.

In a preferred embodiment, the topical compositions according to the present invention comprise as oil soluble UV-filter substances only butyl methoxydibenzoylmethane, octocrylene and polysilicone-15.

In the light protective preparations according to the present invention the amount butyl methoxydibenzoylmethane is preferable selected in the range of about 2 to 8 wt.-%, in particular in the range of about 2.5 to 6 wt.-%, most particular in the range of about 3 to 5 wt.-%, the amount octocrylene is preferable selected in the range of about 2 to 15 wt.-%, in particular in the range of about 4 to 10 wt.-%, most particular in the range of about 6 to 9 wt.-% and the amount of polysilicone-15 is preferable selected in the range of about 0.1 to 5 wt.-%, in particular in the range of about 0.5 to 4 wt.-%, most particular in the range of about 1 to 3 wt.-%, based on the total weight of the topical composition.

In a further advantageous embodiment the light protective preparations comprise next to the at least one oil soluble UV-filter substance further at least one water soluble and/or inorganic UV-filter substance.

Preferably the water soluble UV-filter substance is selected from the group consisting of 2-phenylbenzimidazol-sulphonic acid or 2,2'-(1,4-phenylene)bis-1*H*-benzimidazole-5,7-disulfonic acid as well as the respective salts thereof. Preferably the water soluble UV-filter substance is 2-phenylbenzimidazol-sulphonic acid (e.g. commercially available as PARSOL® HS at DSM Nutritional Products Ltd).

The total amount of the water soluble UV-filter substance(s) in the topical composition according to the invention is preferably selected in the range of about 0.1 to 5 wt.-%, more preferably in the range of about 0.5 to 4 wt.-%, most preferably in the range of 1 to 3 wt.-% based on the total weight of the topical composition.

Inorganic UV-filter substances encompass UV-light absorbing pigments such as e.g. microparticulated Zink oxide or Titanium dioxide. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art. In all embodiment of the present invention, preferably the inorganic UV-filter substance is titanium dioxide having an inner silica and an outer dimethicone coating which is commercially available as PARSOL® TX at DSM Nutritional Products Ltd) is used.

The total amount of the inorganic UV-filter substance(s) in the topical composition according to the invention is preferably selected in the range of about 0.1 to 10 wt.-%, more preferably in the range of about 0.5 to 8 wt.-%, most preferably in the range of 2 to 5 wt.-% based on the total weight of the topical composition.

In particular preferred embodiment the invention relates to topical compositions comprising glycerin, microcrystalline cellulose a, silica beads having a particle size Dᵥ0 of greater 0.3 µm, a Dᵥ100 of less than 35 µm, a Dv50 selected in the range of 9 to 15 µm and an oil absorption capacity selected in the range of 1.2-2.5 cc/g, octocrylene, butyl methoxydibenzoylmethane, polysilicone-15, 2-phenylbenzimidazol-sulphonic acid and titanium dioxide coated having an inner silica and an outer dimethicone coating. In these composition it is further preferred that the amount of octocrylene is selected in the range of 5 to 10 wt.-%, the amount of butyl methoxydibenzoylmethane is selected in the range of 3-5 wt.-%, the amount of polysilicone-15 is selected in the range of 1-3 wt.-% and the amount of coated titanium dioxide is selected in the range of 2-4 wt.-%, based on the total weight of the composition.

The term "topical" as used herein is understood to mean external application to keratinous substances, which are in particular the skin, scalp, eyelashes, eyebrows, nails, mucous membranes and hair.

As the compositions according to the invention are intended for topical application, they comprise a physiologically acceptable medium, that is to say a medium compatible with keratinous substances, such as the skin, mucous membranes, and keratinous fibers. In particular the physiologically acceptable medium is a cosmetically acceptable carrier.

The term cosmetically acceptable carrier refers to all carriers and/or excipients and/or diluents conventionally used in cosmetic compositions.

Preferred topical compositions according to the invention are skin care preparations.

Examples of skin care preparations are, in particular, light protective preparations (sunscreens), anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), as well as skin lightening preparations as well as BB and CC Creams.

In a particular embodiment the topical compositions according to the invention are skin care preparations, such as (body) milks, lotions, hydrodispersions, foundations, creams, creamgels, serums, toners or gels.

The topical compositions according to the present invention may be in the form of a suspension or dispersion in solvents or fatty substances, or alternatively in the form of an emulsion or micro emulsion (in particular of oil-in-water (O/W) or water-in-oil (W/O)type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

In one embodiment, the topical compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the topical composition according to the invention is an O/W emulsion, then it contains advantageously at least one O/W- or Si/W-emulsifier selected from the list of PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, Glyceryl Stearate (and) PEG-100 Stearate , PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate, Steareth-2, Steareth-12, Oleth-2, Ceteth-2, Laureth-4, Oleth-10, Oleth-10/Polyoxyl 10 Oleyl Ether, Ceteth-10, Isosteareth-20, Ceteareth-20, Oleth-20, Steareth-20, Steareth-21, Ceteth-20, Isoceteth-20, Laureth-23, Steareth-100, Glyceryl Stearate Citrate, Glyceryl Stearate SE (self-emulsifying), stearic acid, salts of stearic acid, polyglyceryl-3-methylglycosedistearate. Further suitable emulsifiers are phosphate esters and the salts thereof such as cetyl phosphate (Amphisol® A), diethanolamine cetyl phosphate (Amphisol®DEA), potassium cetyl phosphate (Amphisol® K), sodiumcetearylsulfat, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, Cetearyl Glucoside, Lauryl Glucoside, Decyl Glucoside, Sodium Stearoyl Glutamate, Sucrose Polystearate and Hydrated Polyisobuten. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof.

The at least one O/W, respectively Si/W emulsifier is preferably used in an amount of 0.5 to 10 wt.-%, in particular in the range of 0.5 to 6 wt.-% such as more in particular in the range of 0.5 to 5 wt.-% such as most in particular in the range of 1 to 4 wt.-%, based on the total weight of the composition.

Particular suitable O/W emulsifiers according to the present invention encompass phosphate esters emulsifier of formula (II) 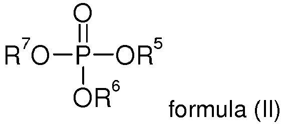 wherein R⁵, R⁶ and R⁷ may be hydrogen, an alkyl of from 1 to 22 carbons, preferably from 12 to 18 carbons; or an alkoxylated alkyl having 1 to 22 carbons, preferably from 12 to 18 carbons, and having 1 or more, preferably from 2 to 25, most preferably 2 to 12, moles ethylene oxide, with the provision that at least one of R⁵, R⁶ and R⁷ is an alkyl or alkoxylated alkyl as previously defined but having at least 6 alkyl carbons in said alkyl or alkoxylated alkyl group.

Monoesters in which R⁵ and R⁶ are hydrogen and R⁷ is selected from alkyl groups of 10 to 18 carbons and alkoxylated fatty alcohols of 10 to 18 carbons and 2 to 12 moles ethylene oxide are preferred. Among the preferred phosphate ester emulsifier are C₈₋₁₀ Alkyl Ethyl Phosphate, C₉₋₁₅ Alkyl Phosphate, Ceteareth-2 Phosphate, Ceteareth-5 Phosphate, Ceteth-8 Phosphate, Ceteth-10 Phosphate, Cetyl Phosphate, C6-10 Pareth-4 Phosphate, C₁₂₋₁₅ Pareth-2 Phosphate, C₁₂₋₁₅ Pareth-3 Phosphate, DEA-Ceteareth-2 Phosphate, DEA-Cetyl Phosphate, DEA-Oleth-3 Phosphate, Potassium cetyl phosphate, Deceth-4 Phosphate, Deceth-6 Phosphate and Trilaureth-4 Phosphate.

A particular suitable O/W emulsifier to be used in the topical compositions according to the invention is potassium cetyl phosphate e.g. commercially available as Amphisol® K at DSM Nutritional Products Ltd Kaiseraugst.

Further suitable O/W emulsifiers are polyethyleneglycol (PEG) esters or diesters such as e.g. [INCI Names] PEG-100 Stearate, PEG-30 Dipolyhydroxystearate, PEG-4 Dilaurate, PEG-8 Dioleate, PEG-40 Sorbitan Peroleate, PEG-7 Glyceryl Cocoate, PEG-20 Almond Glycerides, PEG-25 Hydrogenated Castor Oil, PEG-7 Olivate, PEG-8 Oleate, PEG-8 Laurate, PEG-60 Almond Glycerides, PEG-20 Methyl Glucose Sesquistearate, PEG-40 Stearate, PEG-100 Stearate, PEG-80 Sorbitan Laurate.

Particularly preferred according to the present invention is PEG-100 Stearate sold under the tradename Arlacel™ 165 (INCI Glyceryl Stearate (and) PEG-100 Stearate) by Croda.

Another particular suitable class of O/W emulsifiers are non-ionic self-emulsifying system derived from olive oil e.g. known as (INCI Name) cetearyl olivate and sorbitan olivate (Chemical Composition: sorbitan ester and cetearyl ester of olive oil fatty acids) sold under the tradename OLIVEM 1000.

In particular embodiment, the invention relates to topical compositions with all the definitions and preferences given herein in the form of O/W emulsions comprising an oily phase dispersed in an aqueous phase in the presence of an O/W emulsifier wherein the O/W emulsifier is potassium cetyl phosphate.

The compositions in form of O/W emulsions according to the invention can be provided, for example, in the form of serum, milk or cream, and they are prepared according to the usual methods. The compositions which are subject-matters of the invention are intended for topical application and can in particular constitute a dermatological or cosmetic composition, for example intended for protecting human skin against the adverse effects of UV radiation (antiwrinkle, anti-ageing, moisturizing, anti-sun protection and the like).

According to an advantageous embodiment of the invention the topical compositions constitute cosmetic composition and are intended for topical application to the skin.

In accordance with the present invention, the compositions according to the invention may comprise further ingredients such as ingredients for skin lightening; tanning prevention; treatment of hyperpigmentation; preventing or reducing acne, wrinkles, lines, atrophy and/or inflammation; chelators and/or sequestrants; anti-cellulites and slimming (e.g. phytanic acid), firming, moisturizing and energizing, self-tanning, soothing, as well as agents to improve elasticity and skin barrier and/or further UV-filter substances and carriers and/or excipients or diluents conventionally used in topical compositions. If nothing else is stated, the excipients, additives, diluents, etc. mentioned in the following are suitable for topical compositions according to the present invention. The necessary amounts of the cosmetic and dermatological adjuvants and additives can, based on the desired product, easily be determined by the skilled person. The additional ingredients can either be added to the oily phase, the aqueous phase or separately as deemed appropriate. The mode of addition can easily be adapted by a person skilled in the art.

The cosmetically active ingredients useful herein can in some instances provide more than one benefit or operate via more than one mode of action.

The topical cosmetic compositions of the invention can also contain usual cosmetic adjuvants and additives, such as preservatives/antioxidants, fatty substances/oils, water, organic solvents, silicones, thickeners, softeners, emulsifiers, sunscreens, antifoaming agents, moisturizers, aesthetic components such as fragrances, surfactants, fillers, sequestering agents, anionic, cationic, nonionic or amphoteric polymers or mixtures thereof, propellants, acidifying or basifying agents, dyes, colorings/colorants, abrasives, absorbents, essential oils, skin sensates, astringents, antifoaming agents, pigments or nanopigments, e.g. those suited for providing a photoprotective effect by physically blocking out ultraviolet radiation, or any other ingredients usually formulated into such compositions. Such cosmetic ingredients commonly used in the skin care industry, which are suitable for use in the compositions of the present invention, are e.g. described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the cosmetic and dermatological adjuvants and additives can - based on the desired product - easily be chosen by a skilled person in this field and will be illustrated in the examples, without being limited hereto.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

The topical compositions according to the invention in general have a pH in the range of 3 to 10, preferably a pH in the range of 4 to 8 and most preferably a pH in the range of 4 to 7.5. The pH can easily be adjusted as desired with suitable acids such as e.g. citric acid or bases such as sodium hydroxide (e.g. as aqueous solution), Triethanolamine (TEA Care), Tromethamine (Trizma Base) and Aminomethyl Propanol (AMP-Ultra PC 2000) according to standard methods in the art.

The amount of the topical composition to be applied to the skin is not critical and can easily be adjusted by a person skilled in the art. Preferably the amount is selected in the range of 0.1 to 3 mg/ cm² skin, such as preferably in the range of 0.1 to 2 mg/ cm² skin and most preferably in the range of 0.5 to 2 mg / cm² skin.

The following examples are provided to further illustrate the compositions and effects of the present invention. These examples are illustrative only and are not intended to limit the scope of the invention in any way.

### Example 1: Oil absorption capacity VALVANCE™TOUCH 210

About 2.0g - 2.2g of the silica beads was weighted in a small beaker glass (20ml) at 23 °C. After addition of 4 to 5 drops of paraffin to the powder, mixing was performed using a spatula, and addition of paraffin is continued until conglomerates of oil and powder have formed. From this point, the paraffin was added one drop at a time and the mixture was then triturated with the spatula. The addition of oil was stopped when the loose and dry powder completely disappeared and a highly viscous white to transparent homogeneous gel was obtained. The oil absorption capacity (cc/g) was then calculated by the volume of paraffin used (in cc) per g of silica beads to be 1.58 cc/g.

### Example 2: Sensory evaluation I

### 2.1 Formulations

**Table 1: Sunscreen SPF 30**

| **Ingredients** | **INCI** | **% w/w** |
|---|---|---|
| PARSOL® 1789 | Butyl Methoxydibenzoylmethane | 4.00 |
| PARSOL® 340 | Octocrylene | 8.00 |
| PARSOL® SLX | Polysilicone-15 | 1.50 |
| PARSOL® TX | Titanium Dioxide & Silica & Dimethicone | 3.00 |
| AMPHISOL® K | Potassium Cetyl Phosphate | 2.00 |
| | Cetearyl Alcohol | 0.80 |
| | C12-15 Alkyl Benzoate | 3.00 |
| | Butylene Glycol Dicaprylate/Dicaprate | 4.00 |
| | Dicaprylyl Carbonate | 3.00 |
| | Phenoxyethanol & Ethylhexyl Glycerin | 1.00 |
| | Glycerin | 5.00 |
| | Xanthan Gum | 0.30 |
| | Microcrystalline Cellulose | 1.20 |
| | Disodium EDTA | 0.10 |
| | Aqua | ad 100 |
| | Tromethamine | 0.25 |
| PARSOL® HS | Phenylbenzimidazole Sulfonic Acid | 1.50 |
| | Tromethamine | 0.75 |
| | Aqua | 10.00 |
| VALVANCE™ *Touch* 210 | Silica | 1.50 |

**Table 2: Sunscreen: SPF 50⁺**

| **Ingredients** | **INCI** | % **w/w** |
|---|---|---|
| PARSOL® 1789 | Butyl Methoxydibenzoylmethane | 5.00 |
| PARSOL® 340 | Octocrylene | 14.00 |
| PARSOL® SLX | Polysilicone-15 | 2.00 |
| PARSOL® TX | Titanium Dioxide & Silica & Dimethicone | 3.00 |
| AMPHISOL® K | Potassium Cetyl Phosphate | 2.00 |
| | Cetearyl Alcohol | 1.20 |
| | C12-15 Alkyl Benzoate | 4.00 |
| | Diisopropyl Sebacate | 8.00 |
| | Phenoxyethanol & Ethylhexyl Glycerin | 1.00 |
| | Glycerin | 5.00 |
| | Xanthan Gum | 0.20 |
| | Microcrystalline Cellulose | 0.60 |
| | Disodium EDTA | 0.10 |
| | Aqua | ad 100 |
| | Tromethamine | 0.25 |
| PARSOL® HS | Phenylbenzimidazole Sulfonic Acid | 2.00 |
| | Tromethamine | 1.00 |
| | Aqua | 10.00 |
| VALVANCE™ *Touch* 210 | Silica | 2.00 |

### 2.2 Sensory evaluation

The formulations as outlined in Table 1 and 2 above were tested in a blind study with a trained sensorial panel consisting of 8 persons under the following conditions:
The evaluation takes part on the inner forearm; the panel leader applies 50 µL of the respective sample.

Evaluator spreads the product within a defined circle of 5 cm diameter using index or middle finger, circular motion, rate of 2 rotations/second. This is the so called rub-out phase. After the rub-out phase the stickiness is assessed relative to a set of standards. The stickiness is scaled on a scale from 0 to 100 in comparison to trained standards. Data are median values. The stickiness values are assessed immediately after application as well as after 20 min. As can be retrieved from table 4, the compositions according to the present invention showed a significantly reduced stickiness directly after application as well as after 20 min compared to commercially available sunscreens (listed in table 3).

**Table 3: Stickiness values for commercially available sunscreens with SPF 30 and 50**

| | **References** | Stickiness | |
|---|---|---|---|
| | | Immediate | After 20 min |
| 1 | Garnier Ambre Solaire UV Sensitive Sonnenschutzmilch SPF 50⁺ | 14 | 10 |
| 2 | Nivea sun Kids Pure & Sensitive Sonnenspray SPF 50⁺ | 17 | 10 |
| 3 | Soltan Once Kids Lotion 50⁺ | 17 | 8 |
| 4. | Ombia Suncare Spray 30 | 15 | 5 |

| | | | |
|---|---|---|---|
| 1, Garnier Ambre Solaire, UV Sensitive Sonnenschutzmilch SPF 50⁺ (Company: L'Oreal; purchased 08/2012 in Germany); INCI: Aqua, Octocrylene, Propylene Glycol, Glycerin, Titanium dioxide, C12-15 Alkyl benzoate, Cyclopentasiloxane, Butyl Methoxydibenzoylmethane, Drometrizole Trisiloxane (Benzotriazol), Isohexadecan, Glyceryl Stearate, Potassium Cetyl Phosphate, VP/Eicosene Copolymer, Tocopherol, Phenoxyethanol, PEG-100 Stearate, Ethylparaben, Triethanolamine, Stearic Acid, Chlorphenesin, Dimethicone, Xanthan Gum, Propylparaben, Terephthalylidene Dicamphor Sulfonic Acid, Acrylates/c10-30 Alkyl Acrylate Crosspolymer, Rosa Canina Fruit Oil, Disodium Edta, Methylparaben., Butylparaben, Aluminum Hydroxide, Palmitic Acid, FIL B50529/1. 2. Nivea sun Kids Pure & Sensitive Sonnenspray SPF 50⁺ (Company: Beiersdorf, purchased 07/2012 in Germany); INCI: Aqua, Glycerin, Octocrylene, Alcohol Denat., Butylene Glycol Dicaprylate/Dicaprate, Butyl Methoxydibenzoylmethane, Bis-Ethylhexyloxyphenol Methoxyphenyl Triazine, Homosalate, Titanium Dioxide, Ceteareth-20, C18-36 Acid Triglyceride, Methylpropanediol, Sodium Phenylbenzimidazole Sulfonate, Diethylhexyl Butamido Triazone, Ethylhexyl Methoxycinnamate, Tocopheryl Acetate, Ethylhexylglycerin, PVP/Hexadecene Copolymer, Acrylates/C10-30 Alkyl Acrylate Crosspolymer, Trimethoxycaprylylsilane, Trisodium EDTA, BHT 3. Soltan Once Kids 8 Hour Play Hypo-allergenic Suncare Lotion SPF 50+ (Company: Boots, purchased 07/2012 in Switzerland) ; INCI: Aqua, C12-15 alkyl benzoate, Methylene bis-benzotriazolyl tetramethylbutylphenol (nano), Butyl methoxydibenzoyl-methane, Butylene glycol, Octocrylene, Tridecyl salicylate, PVP/eicosene copolymer, Bis-ethylhexyloxyphenol methoxyphenyl triazine, C18-36 acid glycol ester, Polyglyceryl-3 methylglucose distearate, Titanium dioxide (nano), Polysilicone-15, Decyl glucoside, Phenoxyethanol, Diethylhexyl butamido triazone, Potassium cetyl phosphate, Parfum, Tocopheryl acetate, Vitis vinifera seed oil, Acrylates/vinyl isodecanoate crosspolymer, Glycerin, Dimethyl oxazolidine, Propylene glycol, Potassium hydroxide, Xanthan gum, Tetrasodium EDTA, Manganese dioxide, Panax ginseng extract, Sodium ascorbyl phosphate, Aminomethyl propanol. 4. Ombia Suncare Spray 30 (Company: Aldi; purchased 07/2012 in Germany); INCI: Aqua, Octocrylene, Alcohol, Dibutyl Adipate, Butyl Methoxydibenzoylemethane, Glyzerin, Titanium Dioxide, C12-15 Alkyl Benzoate, VP/Eicosene, Copolymer, Diethylhexyl, Methoxyphenyl, Triazine, Bisabolol, Acrylates/C10-30 Alkyl Acrylate Crosspolymer, Disodium Edta, Ethylhexyglyerin, Silica, Dimethicone. | | | |

**Table 4: Stickiness values for topical compositions according to the present invention**

| **Standard** | Stickiness | |
|---|---|---|
| | Immediate | After 20 min |
| Sunscreen SPF 30 (Table 1) | 6 | 1 |
| Sunscreen SPF 50+ (Table 2) | 6 | 2 |

As can be retrieved from table 4, the topical compositions according to the present invention exhibit a significantly reduced stickiness directly after application as well as after 20 min compared to common market products.

### Example 3: Sensory evaluation II

The formulations as outlined in Table 4 were tested in a blind study with a trained sensorial panel consisting of 8 persons under the following conditions:
The evaluation takes part on the inner forearm; the panel leader applies 50 µL of the respective sample.

Evaluator spreads the product within a defined circle of 5 cm diameter using index or middle finger, circular motion, rate of 2 rotations/second. This is the so called rub-out phase. During this rub-out phase the rotations are counted until the product starts to absorb into skin. The amount of rotations gives a clear number how long a cosmetic product needs to absorb. The absorbency is scaled on a scale from 0 to 100 in comparison to trained standards. The smaller the value the faster absorbs the product. After the rub-out phase the stickiness is assessed relative to a set of standards. The stickiness is scaled on a scale from 0 to 100 in comparison to trained standards. Data are median values. The stickiness values are assessed after 20 min. As can be retrieved from table 4, the addition of microcrystalline wax, respectively microcrystalline cellulose leads to a significantly reduction in the stickiness after 20 min compared to the base and to the reference comprising the silica beads. Furthermore, the absorbency is also significantly improved by the addition of microcrystalline wax, respectively microcrystalline cellulose. In addition, all samples according to the invention exhibit an improved dry touch compared to the base as well as to the reference.

**Table 5**

| | | | **Base** | **Ref** | **Inv 1** | **Ref 2** |
|---|---|---|---|---|---|---|
| | **Ingredients** | **INCI Name** | **% w / w** | | | |
| A | Water dem. | Aqua | Ad 100 | Ad 100 | Ad 100 | Ad 100 |
| | Glycerin 1,23 (86.5%) Ph. Eur. | Glycerin | 11.60 | 11.60 | 11.60 | 11.60 |
| | Avicel PC611 | M icrocrystalline cellulose | - | - | 2.50 | - |
| | Kahlwax 1847 | Hydrogenated microcrystalline wax | - | - | - | 2.50 |
| B | AM PHISOL® K | Potassium cetyl phosphate | 1.50 | 1.50 | 1.50 | 1.50 |
| | Lanette O | Cetearyl alcohol | 3.45 | 3.45 | 3.45 | 3.45 |
| | Myritol 318 | Caprylic/ capric triglyceride | 8.00 | 8.00 | 8.00 | 8.00 |
| | Isopropylpalmitate | Isopropyl palmitate | 8.00 | 8.00 | 8.00 | 8.00 |
| | PARSOL®340 | Octocrylene | - | - | - | - |
| | | | | | | |
| C | VALVANCE Touch 210 | Silica | - | 3.00 | 3.00 | 3.00 |
| | | | | | | |
| D | Phenonip XB | | 0.50 | 0.50 | 0.50 | 0.50 |
| | Stickiness after 20 min | | 1.78 | 0.84 | 0.48 | 0.63 |
| | %reduction in stickiness vs. Base | | | 52.8% | 73.0% | 64.6% |
| | %reduction in stickiness vs. Ref | | | | 43% | 25% |
| | Absorbency | | 90 | 93 | 70 | 74 |
| | %improvement in absorbency vs Base | | | -3% | 22% | 18% |
| | %improvement in absorbency vs Ref | | | | 25% | 20% |

### Example 4: Sunscreen: SPF 50

| | Tradename | INCI | Wt.-% |
|---|---|---|---|
| A | PARSOL 1789 | Butyl Methoxydibenzoylmethane | 3.00 |
| | PARSOL 340 | Octocrylene | 2.70 |
| | PARSOL HMS | Homosalate | 15.00 |
| | PARSOL EHS | Ethylhexyl Salicylate | 5.00 |
| | PARSOL SLX | Polysilicone-15 | 0.90 |
| | PARSOL MCX | Ethylhexyl Methoxycinnmate | 3.00 |
| | Eusolex 4360 | Benzophenone-3 | 5.00 |
| | Amphisol K | Potassium Cetyl Phosphate | 2.00 |
| | Lanette O | Cetearyl Alcohol | 1.20 |
| | Paracera M | Microcrystalline Wax | 2.00 |
| | SilCare Silicone 41M15 | Caprylyl Methicone | 2.00 |
| | BHT | BHT | 0.05 |
| | Euxyl PE 9010 | Phenoxyethanol & Ethylhexyl Glycerin | 1.00 |
| B | Glycerin 86.5% | Glycerin | 5.00 |
| | Keltrol CG SFT | Xanthan Gum | 0.20 |
| | Avicel PC 611 | Microcrystalline Cellulose | 0.60 |
| | Edeta BD | Disodium EDTA | 0.10 |
| | Water demin. | Aqua | Ad 100 |
| D | Allianz OPT | Acrylates (and) C12-22 Alkylmethacrylate Copolymer | 2.00 |
| | VALVANCE™ *Touch* 210 | Silica | 2.00 |

## Claims

1. A topical composition comprising glycerin, microcrystalline cellulose and silica beads having a particle size Dᵥ0 of greater 0.3 µm, a Dᵥ100 of less than 35 µm, a Dv50 selected in the range of 9 to 15 µm determined by a Coulter LS13320 or Malvern Mastersizer 2000 and an oil absorption capacity selected in the range of 1.2-2.5 cc/g, **characterized in that** the composition is an oil-in-water (O/W) emulsion comprising an oily phase dispersed in an aqueous phase in the presence of a potassium cetyl phosphate.

2. The topical composition according to claim 1, **characterized in that** the amount glycerin is selected in the range of 1 to 20 wt.-%, more preferably in the range of 3 to 15 wt.-%, and most preferably in the range of 5 to 10 wt.-%, based on the total weight of the composition.

3. The topical composition according to claim 1 or 2, **characterized in that** the amount of the silica beads is selected in the range of 0.5 to 10 wt.-%, preferably in the range of 1 to 5 wt.-%, and most preferably in the range of 2 to 4 wt.-%, based on the total weight of the composition.

4. The topical composition according to any one of claims 1 to 3, **characterized in that** the total amount of microcrystalline cellulose is selected in the range of 0.1 to 10 wt.-%, more preferably in the range of 0.5 to 5 wt.-%, based on the total weight of the composition.

5. The topical composition according to any one of claims 1 to 4, **characterized in that** no microcrystalline wax is present in the topical composition.

6. The topical composition according to any one of claims 1 to 5, **characterized in that** the oil absorption capacity is selected in the range of 1.2 to 2.0 cc/g and preferably in the range of 1.3 to 1.8 cc/g.

7. The topical composition according to any one of claims 1 to 6, **characterized in that** the topical composition further comprises at least one oil soluble UV-filter substance.

8. The topical compositions according to claim 7, **characterized in that** the at least one oil soluble UV-filter substance is selected from the group consisting of butyl methoxydibenzoylmethane, octocrylene, polysilicone-15 as well as mixtures thereof.

9. The topical composition according to claims 7 and 8, **characterized in that** the total amount of the at least one additional oil soluble UV-filter substance is selected in the range of 0.1 to 25 wt.-%, preferably in the range of 1 to 20 wt.-%, most preferably in the range of 5 to 15 wt.-%, based on the total weight of the topical composition

10. The topical composition according to any one of claims 7 to 9, **characterized in that** the composition further comprises at least one inorganic or water soluble UV-filter substance as well as mixtures thereof.

11. The topical composition according to claim 10, **characterized in that** the water soluble UV-filter substance is 2-phenylbenzimidazol-sulphonic acid and the inorganic UV-filter substance is titanium dioxide having an inner silica and an outer dimethicone coating.

12. Use of silica beads having a particle size Dᵥ0 of greater than 0.3 µm, a Dᵥ100 of less than 35 µm, and a Dᵥ50 selected in the range of 9 to 15 µm and an oil absorption capacity selected in the range of 1.2-2.5 cc/g in combination with microcrystalline cellulose - to reduce the stickiness of a topical composition comprising glycerin, , **characterized in that** the composition is an oil-in-water (O/W) emulsion comprising an oily phase dispersed in an aqueous phase in the presence of a potassium cetyl phosphate.

13. A method to reduce the stickiness of a topical composition comprising glycerin, said method comprising the step of adding to the topical composition silica beads having a particle size Dᵥ0 of greater than 0.3 µm, a Dᵥ100 of less than 35 µm, a Dv50 selected in the range of 9 to 15 µm and an oil absorption capacity selected in the range of 1.2-2.5 cc/g in combination with microcrystalline cellulose, **characterized in that** the composition is an oil-in-water (O/W) emulsion comprising an oily phase dispersed in an aqueous phase in the presence of a potassium cetyl phosphate.

## Patentansprüche

1. Topische Zusammensetzung, umfassend Glycerin, mikrokristalline Cellulose und Siliciumdioxidperlen mit einer Partikelgröße Dᵥ0 von größer als 0,3 µm, einem Dᵥ100 von kleiner als 35 µm, einem Dᵥ50 ausgewählt aus dem Bereich von 9 bis 15 µm, bestimmt durch ein Coulter LS13320 oder Malvern Mastersizer 2000, und einer Ölabsorptionskapazität ausgewählt aus dem Bereich von 1,2-2,5 cc/g, **dadurch gekennzeichnet, dass** die Zusammensetzung eine Ölin-Wasser(O/W)-Emulsion ist, die eine ölige Phase in einer wässrigen Phase dispergiert in Gegenwart eines Kaliumcetylphosphats umfasst.

2. Topische Zusammensetzung gemäß Anspruch 1, **dadurch gekennzeichnet, dass** die Menge an Glycerin ausgewählt ist aus dem Bereich von 1 bis 20 Gew.-%, bevorzugter aus dem Bereich von 3 bis 15 Gew.-% und höchst bevorzugt aus dem Bereich von 5 bis 10 Gew.-%, bezogen auf das Gesamtgewicht der Zusammensetzung.

3. Topische Zusammensetzung gemäß Anspruch 1 oder 2, **dadurch gekennzeichnet, dass** die Menge an Siliciumdioxidperlen ausgewählt ist aus dem Bereich von 0,5 bis 10 Gew.-%, vorzugsweise aus dem Bereich von 1 bis 5 Gew.-% und höchst bevorzugt aus dem Bereich von 2 bis 4 Gew.-%, bezogen auf das Gesamtgewicht der Zusammensetzung.

4. Topische Zusammensetzung gemäß einem der Ansprüche 1 bis 3, **dadurch gekennzeichnet, dass** die Gesamtenge an mikrokristalliner Cellulose ausgewählt ist aus dem Bereich von 0,1 bis 10 Gew.-%, bevorzugter aus dem Bereich von 0,5 bis 5 Gew.-%, bezogen auf das Gesamtgewicht der Zusammensetzung.

5. Topische Zusammensetzung gemäß einem der Ansprüche 1 bis 4, **dadurch gekennzeichnet, dass** in der topischen Zusammensetzung kein mikrokristallines Wachs vorhanden ist.

6. Topische Zusammensetzung gemäß einem der Ansprüche 1 bis 5, **dadurch gekennzeichnet, dass** die Ölabsorptionskapazität ausgewählt ist aus dem Bereich von 1,2 bis 2,0 cc/g, bevorzugter aus dem Bereich von 1,3 bis 1,8 cc/g.

7. Topische Zusammensetzung gemäß einem der Ansprüche 1 bis 6, **dadurch gekennzeichnet, dass** die topische Zusammensetzung ferner wenigstens einen öllöslichen UV-Filterstoff umfasst.

8. Topische Zusammensetzung gemäß Anspruch 7, **dadurch gekennzeichnet, dass** der wenigstens eine öllösliche UV-Filterstoff ausgewählt ist aus der Gruppe bestehend aus Butylmethoxydibenzoylmethan, Octocrylen, Polysilicon-15 und Gemischen davon.

9. Topische Zusammensetzung gemäß Ansprüchen 7 und 8, **dadurch gekennzeichnet, dass** die Gesamtmenge des wenigstens einen zusätzlichen öllöslichen UV-Filterstoffs ausgewählt ist aus dem Bereich von 0,1 bis 25 Gew.-%, vorzugsweise aus dem Bereich von 1 bis 20 Gew.-%, höchst bevorzugt aus dem Bereich von 5 bis 15 Gew.-%, bezogen auf das Gesamtgewicht der Zusammensetzung.

10. Topische Zusammensetzung gemäß einem der Ansprüche 7 bis 9, **dadurch gekennzeichnet, dass** die Zusammensetzung ferner wenigstens einen anorganischen oder wasserlöslichen UV-Filterstoff oder Gemische davon umfasst.

11. Topische Zusammensetzung gemäß Anspruch 10, **dadurch gekennzeichnet, dass** der wasserlösliche UV-Filterstoff 2-Phenylbenzimidazolsulfonsäure ist und der anorganische UV-Filterstoff Titandioxid mit einer innenliegenden Siliciumdioxid und einer äußeren Dimethiconbeschichtung ist.

12. Verwendung von Siliciumdioxidperlen mit einer Partikelgröße Dᵥ0 von größer als 0,3 µm, einem Dᵥ100 von kleiner als 35 µm, einem Dᵥ50 ausgewählt aus dem Bereich von 9 bis 15 µm und einer Ölabsorptionskapazität ausgewählt aus dem Bereich von 1,2-2,5 cc/g in Kombination mit mikrokristalliner Cellulose - zum Verringern der Klebrigkeit einer topischen Zusammensetzung, die Glycerin umfasst, **dadurch gekennzeichnet, dass** die Zusammensetzung eine Öl-in-Wasser(O/W)-Emulsion ist, die eine ölige Phase in einer wässrigen Phase dispergiert in Gegenwart eines Kaliumcetylphosphats umfasst.

13. Verfahren zum Verringern der Klebrigkeit einer topischen Zusammensetzung, die Glycerin umfasst, wobei das Verfahren den Schritt des Zugebens von Siliciumdioxidperlen mit einer Partikelgröße Dᵥ0 von größer als 0,3 µm, einem Dᵥ100 von kleiner als 35 µm, einem Dᵥ50 ausgewählt aus dem Bereich von 9 bis 15 µm und einer Ölabsorptionskapazität ausgewählt aus dem Bereich von 1,2-2,5 cc/g in Kombination mit mikrokristalliner Cellulose zu der topischen Zusammensetzung umfasst, **dadurch gekennzeichnet, dass** die Zusammensetzung eine Öl-in-Wasser(O/W)-Emulsion ist, die eine ölige Phase in einer wässrigen Phase dispergiert in Gegenwart eines Kaliumcetylphosphats umfasst.

## Revendications

1. Composition topique comprenant du glycérol, de la cellulose microcristalline et des billes de silice ayant une taille de particules Dᵥ0 supérieure à 0,3 µm, une Dᵥ100 inférieure à 35 µm, une Dᵥ50 choisie dans la plage allant de 9 à 15 µm, déterminée à l'aide d'un Coulter LS13320 ou d'un Malvern Mastersizer 2000, et une capacité d'absorption d'huile choisie dans la plage de 1,2-2,5 cc/g, **caractérisée en ce que** la composition est une émulsion huile-dans-eau (H/E) comprenant une phase huileuse dispersée dans une phase aqueuse en présence d'un cétylphosphate de potassium.

2. Composition topique selon la revendication 1, **caractérisée en ce que** la quantité de glycérol est choisie dans la plage allant de 1 à 20% en poids, plus préférablement dans la plage allant de 3 à 15% en poids, et tout préférablement dans la plage allant de 5 à 10% en poids, sur la base du poids total de la composition.

3. Composition topique selon la revendication 1 ou 2, **caractérisée en ce que** la quantité de billes de silice est choisie dans la plage allant de 0,5 à 10% en poids, préférablement dans la plage allant de 1 à 5% en poids, et tout préférablement dans la plage allant de 2 à 4% en poids, sur la base du poids total de la composition.

4. Composition topique selon l'une quelconque des revendications 1 à 3, **caractérisée en ce que** la quantité totale de cellulose microcristalline est choisie dans la plage allant de 0,1 à 10% en poids, plus préférablement dans la plage allant de 0,5 à 5% en poids, sur la base du poids total de la composition.

5. Composition topique selon l'une quelconque des revendications 1 à 4, **caractérisée en ce qu'**aucune cire microcristalline n'est présente dans la composition topique.

6. Composition topique selon l'une quelconque des revendications 1 à 5, **caractérisée en ce que** la capacité d'absorption d'huile est choisie dans la plage allant de 1,2 à 2,0 cc/g et préférablement dans la plage allant de 1,3 à 1,8 cc/g.

7. Composition topique selon l'une quelconque des revendications 1 à 6, **caractérisée en ce que** la composition topique comprend en outre au moins une substance de filtre anti-UV liposoluble.

8. Composition topique selon la revendication 7, **caractérisée en ce que** la au moins une substance de filtre anti-UV liposoluble est choisie dans le groupe constitué par le butylméthoxydibenzoylméthane, l'octocrylène, la polysilicone-15, ainsi que des mélanges de ceux-ci.

9. Composition topique selon les revendications 7 et 8, **caractérisée en ce que** la quantité totale de la au moins une substance de filtre anti-UV liposoluble supplémentaire est choisie dans la plage allant de 0,1 à 25% en poids, préférablement dans la plage allant de 1 à 20% en poids, tout préférablement dans la plage allant de 5 à 15% en poids, sur la base du poids total de la composition topique.

10. Composition topique selon l'une quelconque des revendications 7 à 9, **caractérisée en ce que** la composition comprend en outre au moins une substance de filtre anti-UV hydrosoluble ou inorganique, ainsi que des mélanges de celle-ci.

11. Composition topique selon la revendication 10, **caractérisée en ce que** la substance de filtre anti-UV hydrosoluble est l'acide 2-phénylbenzimidazolsulfonique et la substance de filtre anti-UV inorganique est le dioxyde de titane ayant un revêtement de silice interne et de diméthicone externe.

12. Utilisation de billes de silice ayant une taille de particules Dᵥ0 supérieure à 0,3 µm, une Dᵥ100 inférieure à 35 µm, et une Dᵥ50 choisie dans la plage allant de 9 à 15 µm, et une capacité d'absorption d'huile choisie dans la plage de 1,2-2,5 cc/g, en combinaison avec de la cellulose microcristalline - afin de réduire le caractère collant d'une composition topique comprenant du glycérol, **caractérisée en ce que** la composition est une émulsion huile-dans-eau (H/E) comprenant une phase huileuse dispersée dans une phase aqueuse en présence d'un cétylphosphate de potassium.

13. Méthode de réduction du caractère collant d'une composition topique comprenant du glycérol, ladite méthode comprenant l'étape consistant à ajouter à la composition topique des billes de silice ayant une taille de particules Dᵥ0 supérieure à 0,3 µm, une Dᵥ100 inférieure à 35 µm, une Dᵥ50 choisie dans la plage allant de 9 à 15 µm, et une capacité d'absorption d'huile choisie dans la plage de 1,2-2,5 cc/g, en combinaison avec de la cellulose microcristalline, **caractérisée en ce que** la composition est une émulsion huile-dans-eau (H/E) comprenant une phase huileuse dispersée dans une phase aqueuse en présence d'un cétylphosphate de potassium.
